# EUROPÄISCHE PATENTANMELDUNG

(11) **EP 1 203 589 A1**
(43) Veröffentlichungstag der Anmeldung: **08.05.2002**
(21) Anmeldenummer: 01123921.7
(22) Anmeldetag: 06.10.2001
(51) Int. Cl.: A61K 47/10

(54) **Verwendung von Terpenalkohol-Ethoxylaten als Solubilisatoren in kosmetischen oder pharmazeutischen Zubereitungen oder konzentraten für Lebensmittelzubereitungen**

(30) Priorität: 27.10.2000 DE 10053512
(71) Anmelder: BASF AKTIENGESELLSCHAFT, 67056 Ludwigshafen (DE)
(72) Erfinder: Zirnstein, Michael, Dr., 69198 Schriesheim (DE); Rock, Tilman C., Dr., 74889 Sinsheim (DE); Kolter, Karl, Dr., 67117 Limburgerhof (DE)

(57) **Zusammenfassung**

Verwendung von Terpenalkoholen mit 10 C-Atomen, die mit 3 bis 10 Mol Ethylenoxid ethoxyliert sind, als Solubilisatoren für schwer wasserlösliche oder schwer wasserdispergierbare Verbindungen in kosmetischen oder pharmazeutischen Zubereitungen oder in Konzentraten für Lebensmittelzubereitungen in Mengen von mindestens 3 Gew.-%, bezogen auf die fertigen Zubereitungen bzw. bezogen auf die Konzentrate.

## Beschreibung

Die Erfindung betrifft die Verwendung von Terpenalkoholen, die mit 3 bis 10 Mol Ethylenoxid ethoxyliert sind als Solubilisatoren in kosmetischen oder pharmazeutischen Zubereitungen oder Konzentraten für Lebensmittelzubereitungen in Mengen von mindestens 3 Gew.-%.

Aus GB 2 297 557 ist bekannt, daß man etherische Öle, z.B. Geraniol durch Alkoxylierung wasserlöslich machen kann, ohne daß die Geruchseigenschaften in großem Maße verloren gehen. Die dort beschriebenen Alkoxylate sollen demnach später als Riechstoffe, d.h. in sehr geringen Mengen, in dort nicht beschriebenen Zubereitungen eingesetzt werden.

Für kosmetische und pharmazeutische Zubereitungen sind Solubilisatoren aus den verschiedensten Verbindungsgruppen in großer Zahl bekannt.

Bei der Herstellung homogener pharmazeutischer oder kosmetischer Zubereitungen hat nämlich die Solubilisierung von hydrophoben Stoffen eine sehr große praktische Bedeutung erlangt.

Unter Solubilisierung ist eine Löslichkeitsverbesserung durch oberflächenaktive Verbindungen zu verstehen, die in der Lage sind, schlecht wasserlösliche oder wasserunlösliche Stoffe in klare, höchstens opaleszierende wäßrige Lösungen zu überführen, ohne daß hierbei die chemische Struktur dieser Stoffe eine Veränderung erfährt.

Die hergestellten Solubilisate sind dadurch gekennzeichnet, daß der schlecht wasserlösliche oder wasserunlösliche Stoff in den Molekülassoziaten der oberflächenaktiven Verbindungen, die sich in wäßriger Lösung bilden - den sogenannten Mizellen - gelöst vorliegt. Die resultierenden Lösungen sind stabile einphasige Systeme, die optisch klar bis opaleszent erscheinen und ohne größeren Energieeintrag hergestellt werden können.

Solubilisatoren können das Aussehen beispielsweise von kosmetischen Formulierungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich folgende Produkte eingesetzt:
- ethoxiliertes (hydriertes) Ricinusöl, (z.B. Cremophor® Marken, Fa. BASF);
- ethoxilierte Sorbitanfettsäureester, (z.B. Tween® Marken, Fa. ICI);
- ethoxilierte Hydroxystearinsäure, (z.B. Solutol® Marken, Fa. BASF).

Diese bekannten Solubilisatoren haben sich zwar bewährt, doch sind sie weiter verbesserungsbedürftig in Bezug auf die Solubilisations- und Anwendungseigenschaften, sowie die physiologische Verträglichkeit.

Es bestand daher die Aufgabe, neue Solubilisatoren vorzuschlagen, die sehr gute Solubilisierungseigenschaften, verbesserte Stabilität, gute physiologische Verträglichkeit und leichte Dosierbarkeit in flüssiger Form vereinen.

Es wurde nun überraschenderweise gefunden, daß Umsetzungsprodukte von C₁₀-Terpenalkoholen mit 3 bis 10 Mol Ethylenoxid, nicht nur selbst gut wasserlöslich sind, sondern überdurchschnittlich solubilisierend für in Wasser schwer lösliche oder schwer dispergierbare Verbindungen wirken, wenn man sie in Mengen von mindestens 3 Gew.-%, bezogen auf die fertigen Zubereitungen bzw. Konzentrate für Zubereitungen einsetzt.

Unter schwer wasserlöslichen oder schwer wasserdispergierbaren Verbindungen, d.s. kosmetische oder pharmazeutische Wirkstoffe, bzw. Lebensmittelzusatzstoffe, werden im allgemeinen Verbindungen verstanden, die eine Löslichkeit von weniger als 5 Gew.-% und insbesondere weniger als 1 Gew.-% in Wasser aufweisen. In Wasser schwer dispergierbare Stoffe sind in der Regel wasserunlösliche oder praktisch wasserunlösliche Stoffe, die nur mit Hilfe eines Solubilisators in eine stabile Dispersion in Wasser oder überwiegend wässrigem Medium überführt werden können.

Für pharmazeutische Zubereitungen soll dabei der Begriff schwer wasserlösliche Wirkstoffe auch die in Wasser wenig löslichen Wirkstoffe gemäß DAB 9 (Deutsches Arzneimittelbuch) einschließen. Dort ist die Einstufung wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10 000 Teilen Lösungsmittel).

Die erfindungsgemäß zu verwendenden Umsetzungsprodukte lassen sich ferner leicht flüssig dosieren, haben eine höhere Hydrolysebeständigkeit als die üblicherweise verwendeten Ester und sind gut physiologisch verträglich. Insbesondere weisen sie eine sehr geringe Hämolyseaktivität auf.

Die erfindungsgemäß zu verwendenden Verbindungen sind z.B. Nerol, Linalool, Menthol, Terpineol und insbesondere Geraniol und Citronellol, die mit 3 bis 10, vorzugsweise 5 bis 7 Mol Ethylenoxid umgesetzt sind.

Die Anwendung der Solubilisatoren erfolgt in Mengen von 3 bis 90, vorzugsweise 3 bis 50 und insbesondere 3 bis 30 Gew.-%, bezogen auf die fertigen Zubereitungen bzw. Konzentrate.

Die Herstellung der erfindungsgemäß zu verwendenden Verbindungen durch Umsetzung mit Ethylenoxid erfolgt in an sich bekannter Weise, wie dies z.B. in N. Schönfeldt, Grenzflächenaktive Ethylenoxid-Addukte, Wissenschaftliche Verlagsgesellschaft mbH, Stuttgart, 1. Auflage (1976) und Ergänzungsband (1984) beschrieben ist.

In der Regel wird der Alkohol in Gegenwart eines Katalysators, vorzugsweise Natrium- oder Kaliumhydroxid (üblicherweise 0,05 bis 5 Gew.-%, vorzugsweise 0,1 bis 0,5 Gew.-% bezogen auf den zu oxethylierenden Alkohol) mit Ethylenoxid bis zum gewünschten Oxethylierungsgrad in inerter Atmosphäre umgesetzt. Der Katalysator kann in Form einer wässrigen Lösung zugegeben werden. Vorteilhaft wird vor der eigentlichen Dosierung des Ethylenoxids der Ansatz entwässert bei 50 bis 110°C, bevorzugt bei 60 bis 90°C und unter Vakuum. Die Oxethylierung von primären Alkoholen wird bei 90 bis 150°C, bevorzugt bei 120 bis 140°C; die Oxethylierung von sekundären Alkoholen bei 120 bis 170°C, bevorzugt bei 140 bis 160°C durchgeführt.

Das Reaktionsprodukt kann optional einer Nachbehandlung unterzogen werden. Hierzu zählen unter anderem die Neutralisation des Katalysators durch Zugabe von Säuren, wie beispielsweise Phosphorsäure, oder Ionenaustauschern oder die Zugabe von Adsorbentien wie Silikate oder Aluminiumoxide. Die Nachbehandlung kann bei Temperaturen von 10 bis 180°C, vorzugsweise 30 bis 100°C, durchgeführt werden und erfolgt unter Rühren über 0,5 bis 12 h. In der Regel schließt sich ein Filtrationsschritt an, gegebenenfalls unter Verwendung eines Filtrierhilfsmittels, wie z.B. Diatomeenerde. Auch eine Kombination dieser Mittel zur Nachbehandlung kann sinnvoll sein.

Als Silikate werden bevorzugt Alkali- oder Erdalkalisilikate, insbesondere Mg-Silikate oder Ca-Silikate, oder Al-Silikate, aber auch Bleicherden eingesetzt. Als Handelsprodukte seien beispielsweise Ambosol, Tonsil FF oder Magnesol genannt.

### Anwendungen:

Durch die vorliegende Erfindung werden wasserlösliche, amphiphile Verbindungen für die Anwendung als Lösungsvermittler für pharmazeutische und kosmetische Zubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe in wäßrigen Systemen zu solubilisieren.

### Solubilisatoren für Kosmetik

Die erfindungsgemäß zu verwendenden Verbindungen können als Solubilisatoren in kosmetischen Formulierungen eingesetzt werden. Besonders eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Weiterhin können die erfindungsgemäß zu verwendenden Verbindungen als Solubilisatoren für in Wasser schwerlösliche oder unlösliche UV-Absorber wie beispielsweise 2-Hydroxy-4-methoxybenzophenon (Uvinul® M 40, Fa. BASF), 2,2',4,4'-Tetrahydroxybenzophenon (Uvinul® D 50), 2,2'-Dihydroxy-4,4'-dimethoxybenzophenon (Uvinul® D49), 2,4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul® T 150), 3-(4-Methoxybenzyliden)-campher (Eusolex® 6300, Fa. Merck), N,N-Dimethyl-4-aminobenzoesäure-2-ethylhexylester (Eusolex® 6007), Salicylsäure-3,3,5-trimethylcyclohexylester, 4-Isopropyldibenzoylmethan (Eusolex® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens eine der erfindungsgemäß zu verwendenden Verbindungen als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol-Basis. Die Verbindung I wird als Solubilisator im Verhältnis von 0,2:1 bis 50:1, bevorzugt 0,5:1 bis 20:1, besonders bevorzugt 1:1 bis 15:1, ganz besonders bevorzugt 2:1 bis 12:1 zum schwerlöslichen kosmetischen Wirkstoff eingesetzt.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholsulfonate, Fettalkoholethersulfate, Fettalkoholethersulfonate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Mundwässern, Haarwässern, Eau de Cologne, Eau de Toilette usw. Verwendung.

### Beschreibung der Solubilisierungsmethode:

Bei der Herstellung der Solubilisate für kosmetische Formulierugen können die erfindungsgemäß zu verwendenden Verbindungen als 100 %ige Substanz oder als wäßrige Lösung eingesetzt werden.

Üblicherweise wird der Solubilisator in Wasser gelöst und mit dem jeweils zu verwendenden schwerlöslichen kosmetischen Wirkstoff z.B. den o.g. etherischen Ölen bzw. Parfümölen intensiv vermischt, beispielsweise mittels eines Magnetrührers.

Es kann aber auch der zu verwendende schwerlösliche kosmetische Wirkstoff in einer Schmelze des Solubilisators gelöst werden und anschließend unter ständigem Rühren mit demineralisiertem Wasser versetzt werden.

### Solubilisatoren für pharmazeutische Anwendungen

Die erfindungsgemäß zu verwendenden Verbindungen eignen sich für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe oder Vitamine sowie Carotinoide enthalten. Insbesondere handelt es sich dabei um wäßrige Lösungen bzw. Solubilisate zur oralen oder parenteralen Applikation, wie z.B. Injektionslösungen zur intravenösen, intramuskulären oder subkutaner oder intraperitonealer Applikation.

Desweiteren eignen sich die erfindungsgemäß zu verwendenden Verbindungen zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die beanspruchten Verbindungen um einen schwerlöslichen Arzneistoff zu verarbeiten.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die Wirkstoffe können dabei aus jedem Indikationsbereich kommen. Als Beispiele seien hier Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Die Verwendung der erfindungsgemäßen Verbindungen als Lösungsvermittler in pharmazeutischen Zubereitungen erfolgt beispielsweise in der Weise, daß der Wirkstoff in dem Solubilisator, gegebenenfalls unter Erwärmen, dispergiert oder gelöst wird und unter Rühren mit Wasser vermischt wird.

Eine andere Herstellvariante ist das Auflösen des Solubilisators in der wäßrigen Phase, gegebenenfalls unter leichtem Erwärmen und das anschließende Lösen des Wirkstoffs in der wäßrigen Solubilisatorlösung. Das gleichzeitige Auflösen von Solubilisator und Wirkstoff in der wäßrigen Phase ist ebenfalls möglich.

Gegenstand der Erfindung sind daher auch pharmazeutische Zubereitungen, die mindestens eine der erfindungsgemäß zu verwendenden Verbindungen als Solubilisatoren enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um parenteral applizierbare Formulierungen handelt.

Der Gehalt an erfindungsgemäßem Solubilisator in der pharmazeutischen Zubereitung liegt, abhängig vom Wirkstoff, im Bereich von 3 bis 90 Gew.-%, bevorzugt 3 bis 50 Gew.-%, besonders bevorzugt 3 bis 30 Gew.-%.

### Solubilisatoren für Lebensmittelzubereitungen

Neben der Anwendung in der Kosmetik und Pharmazie eignen sich die erfindungsgemäß zu verwendenden Verbindungen auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiel seien klare, mit Carotinoiden gefärbte Getränke genannt.

Gegenstand der Erfindung sind daher auch Konzentrate (Masterbatches) für Lebensmittelzubereitungen, wobei die Konzentrate mindestens eine der erfindungsgemäß zu verwendenden Verbindungen als Solubilisatoren in Mengen von mindestens 3 Gew.-% enthalten. Bevorzugt sind solche Konzentrate, die neben dem Solubilisator ein in Wasser schwerlösliches oder wasserunlösliches Vitamin oder Carotinoid enthalten.

### Beispiele

### Herstellung der Ethoxylate

### Beispiel 1

### Citronellol + 8 EO

Eine Mischung von 265,5 g (1,70 mol) Citronellol und 0,53 g Kaliumhydroxid wurde bei 70°C im Vakuum (< 10 mbar) 1 h entwässert. In einem mit Stickstoff inertisierten Autoklaven wurden unter Rühren bei 140°C 600 g (13,6 mol) Ethylenoxid innerhalb von 3,5 h zudosiert. Hierbei wurde ein Überdruck von bis zu 7 bar erreicht. Nach beendeter Ethylenoxid-Dosierung wurde noch 1 h bei 140°C gerührt. Eine weitere Stunde wurde bei 70°C und < 10 mbar Vakuum gerührt, mit 3,54 g Ambosol ® (Hoechst) versetzt, eine Stunde bei 80°C gerührt und druckfiltriert. Ausbeute: 850 g (98,2 %), OHZ = 109 mg KOH/g.

### Beispiel 2

### Citronellol + 5,5 EO

Eine Mischung von 330 g (2,12 mol) Citronellol und 0,66 g Kaliumhydroxid wurde bei 60 bis 70°C im Vakuum (≥ 1 mbar) bis auf einen Restwassergehalt von 0,04 % entwässert. Zu 312 g (2,00 mol) dieses Citronellol-Ansatzes wurden in einem mit Stickstoff inertisierten Autoklaven unter Rühren bei 130°C 471 g (10,7 mol) Ethylenoxid innerhalb von 4 h zudosiert. Hierbei wurde ein Überdruck von bis zu 7 bar erreicht. Nach beendeter Ethylenoxid-Dosierung wurde noch 1 h bei 130°C gerührt. Eine weitere Stunde wurde bei 70°C und ≥ 1 mbar Vakuum gerührt, mit 4,40 g Ambosol versetzt, eine Stunde bei 80°C gerührt und druckfiltriert. Ausbeute: 772 g (98,6 %), OHZ = 142 mg KOH/g.

### Beispiel 3

### Geraniol + 9 EO

Eine Mischung von 277,6 g (1,80 mol) Citronellol und 0,56 g Kaliumhydroxid wurde bei 70°C im Vakuum (< 10 mbar) 1 h entwässert. In einem mit Stickstoff inertisierten Autoklaven wurden unter Rühren bei 140°C 717 g (16,3 mol) Ethylenoxid innerhalb von 4,5 h zudosiert. Hierbei wurde ein Überdruck von bis zu 7 bar erreicht. Nach beendeter Ethylenoxid-Dosierung wurde noch 1,5 h bei 140°C gerührt. Eine weitere Stunde wurde bei 70°C und < 10 mbar Vakuum gerührt, mit 3,31 g Ambosol versetzt, eine Stunde bei 70°C gerührt und druckfiltriert. Ausbeute: 1005 g (quant.),
OHZ = 100 mg KOH/g.

### Verwendung

### Beispiel 4

### (Piroxicam-Injektionslösung)

2,5 g Piroxicam wurden unter Rühren in eine Mischung aus 200 g Citronellolethoxylat mit 9 Mol Ethylenoxid und 2,0 g Benzylalkohol eingetragen. Anschließend wurde auf 50°C erwärmt und solange gerührt bis das Piroxicam vollständig in Lösung gegangen war. Dann wurde die klare Lösung langsam mit 795,4 g Wasser für Injektionszwecke versetzt und auf Raumtemperatur abgekühlt. Nach einer Sterilfiltration über ein 0,22 µm-Filter wurde die klare Lösung unter sterilen Bedingungen in 10 ml-Ampullen abgefüllt und die Ampullen zugeschmolzen.

### Beispiel 5

### (Vitamin E-Acetat Trinklösung)

25 g Vitamin E-Acetat wurden unter Rühren bei 60°C in 200 g Citronellolethoxylat mit 6 Mol Ethylenoxid gelöst. Diese Lösung wurde anschließend langsam mit 775 g einer separat hergestellten Lösung von 200 g Sorbit, 1,0 g Aspartame und 2,0 g Mandarinenaroma in 572,0 g gereinigtem Wasser versetzt. Nach Abkühlung auf Raumtemperatur wurde die Vitamin E-Acetat-Trinklösung in 100 ml-Flaschen mit Schraubverschluß abgefüllt.

### Beispiel 6

### (Diazepam-Granulat)

10.0 g Diazepam wurden bei 50°C in 200,0 g einer Mischung aus 6 Gewichtsteilen Geraniolethoxylat mit 7 Mol Ethylenoxid und 4 Gewichtsteilen Ethanol gelöst. In einem Lödige-Pflugscharmischer wurde diese Lösung auf eine Pulvermischung bestehend aus 1000,0 g Lactose, 850,0 g mikrokristalline Cellulose (Avicel® PH 102) und 20,0 g hochdisperser Kieselsäure (Aerosil® OX 50) gesprüht. Anschließend wurde das Granulat in einem Hordentrocknerschrank bei 30°C abgetrocknet und in Sachets ä 2,0 g abgefüllt.

### Beispiel 7

### (Nipedipin-Kapseln)

40,0 g Nifedipin wurden unter Lichtausschluß und unter Rühren in einer Mischung aus 200,0 g Citronellolethoxylat mit 6 Mol Ethylenoxid, 400,0 g Polyethylenglykol 6000 und 360,0 g Polyoxyethylen-Polyoxypropylen-Blockpolymer (Lutrol® F 68) bei 60°C gelöst. Diese Lösung wurde in Hartgelatinekapseln der Größe 00 gefüllt, wobei jeweils 500 mg eindosiert wurden. Nach Erkalten des Kapselinhaltes wurden Ober- und Unterteil der Kapsel durch Banderolieren mit 30 %iger Gelatinelösung dicht verschlossen.

### Beispiele 8 bis 13 (Vergleichsbeispiele 8 bis 10)

Die Terpenalkohol-Ethoxylate wurden jeweils auf 65°C erwärmt und der Arzneistoff hinzugegeben. Danach wurde der Phosphatpuffer pH 7,0 (USP XXIII; 34,0 g Kaliumdihydrogenphosphat und 145,5 ml 1N-Natronlauge in 4000,0 g demineralisiertem Wasser gelöst und mit demineralisiertem Wasser ad 5,0 1 aufgefüllt) in kleinen Anteilen bis zur Einstellung einer Solubilisatorkonzentration von 20 Gew.-%, bezogen auf die fertige Lösung, hinzugefügt. Es wurde bei Raumtemperatur gerührt bis die Sättigungskonzentration des Arzneistoffs erreicht war. Die erhaltenen Sättigungskonzentrationen in Gew.-% sind in der folgenden Tabelle angegeben.

**Tabelle**

| Bsp. | Verbindung | Sulfathiazol | 17-β-Estradiol | Clotrimazol |
|---|---|---|---|---|
| 8 | Phosphatpuffer pH 7,0 allein | 0,07 | 0,0 | 0,0 |
| 9 | Sorbitanfettsäureester (Tween® 80) | 0,7 | 0,09 | 0,03 |
| 10 | Ethoxyliertes Ricinusöl (Cremophor® EL) | 0,7 | 0,06 | 0,01 |
| 11 | Citronellol-Ethoxylat mit 8 EO | 0,95 | 0,23 | 0,39 |
| 12 | Citronellol-Ethoxylat mit 6 EO | 0,78 | 0,30 | - |
| 13 | Citronellol-Ethoxylat mit 9 EO | - | 0,14 | - |

### Beispiel 14

### (Sonnenschutzmittel)

25 g Citronellol-Ethoxylat mit 6 EO wurden auf 60°C erwärmt und darin 2,5 g Uvinul® T 150 unter Rühren gelöst. Anschließend wurde eine auf 60°C erwärmte Mischung von 62,5 g Aqua bidest, 5 g Glycerol und 5 g 1,2-Propylenglykol vorsichtig unter Rühren hinzugetropft. Es entstand eine klare Lösung, die nach Erkalten auf Raumtemperatur in ein geeignetes Behältnis abgefüllt wurde.

## Patentansprüche

1. Verwendung von Terpenalkoholen mit 10 C-Atomen, die mit 3 bis 10 Mol Ethylenoxid ethoxyliert sind, als Solubilisatoren für schwer wasserlösliche oder schwer wasserdispergierbare Verbindungen in kosmetischen oder pharmazeutischen Zubereitungen oder Konzentraten für Lebensmittelzubereitungen, in Mengen von mindestens 3 Gew.-%, bezogen auf die fertigen kosmetischen und pharmazeutischen Zubereitungen, bzw. bezogen auf die Konzentrate für Lebensmittelzubereitungen.

2. Verwendung gemäß Anspruch 1, **dadurch gekennzeichnet, daß** das C₁₀-Terpenalkoholethoxylat Citronellol, umgesetzt mit 5 bis 7 Mol Ethylenoxid, ist.

3. Verwendung gemäß Anspruch 1, **dadurch gekennzeichnet, daß** das C₁₀-Terpenalkoholethoxylat Geraniol, umgesetzt mit 5 bis 7 Mol Ethylenoxid, ist.

4. Kosmetische oder pharmazeutische Zubereitungen, enthaltend mindestens 3 Gew.-% mit 3 bis 10 Mol Ethylenoxid alkoxylierte C₁₀-Terpenalkohole als Solubilisatoren für schwer wasserlösliche oder schwer wasserdispergierbare Wirkstoffe.

5. Kosmetische oder pharmazeutische Zubereitungen gemäß Anspruch 4, enthaltend 3 bis 50 Gew.-%, bezogen auf die fertigen Zubereitungen, mit 3 bis 10 Mol Ethylenoxid alkoxilierte C₁₀-Terpenalkohole als Solubilisatoren für schwer wasserlösliche oder schwer wasserdispergierbare Wirkstoffe.

6. Kosmetische oder pharmazeutische Zubereitungen gemäß Anspruch 4, **dadurch gekennzeichnet, daß** das C₁₀-Terpenalkoholethoxylat Citronellol, umgesetzt mit 5 bis 7 Mol Ethylenoxid, ist.

7. Kosmetische oder pharmazeutische Zubereitungen gemäß Anspruch 4, **dadurch gekennzeichnet, daß** das C₁₀-Terpenalkoholethoxylat Geraniol, umgesetzt mit 5 bis 7 Mol Ethylenoxid, ist.

8. Konzentrate für Lebensmittelzubereitungen, enthaltend 3 bis 50 Gew.-% mit 3 bis 10 Mol Ethylenoxid alkoxylierte C₁₀-Terpenalkohole als Solubilisatoren für schwer wasserlösliche oder schwer wasserdispergierbare Lebensmittelzusatzstoffe.
